# EUROPEAN PATENT APPLICATION

(11) **EP 3 711 807 A1**
(43) Date of publication of application: **23.09.2020**
(21) Application number: 19164321.2
(22) Date of filing: 21.03.2019
(51) Int. Cl.: A61M 25/00, A61M 25/01

(54) **URINARY CATHETER ASSEMBLY**

(71) Applicant: Dentsply IH AB, 431 21 Mölndal (SE)
(72) Inventor: ERIKSSON, Karsten, 475 45 FOTÖ (SE); SANDBERG, Michael, 436 39 ASKIM (SE)
(74) Representative: AWA Sweden AB

(57) **Abstract**

A urinary catheter assembly comprises a urinary catheter 1 and a tubular casing 2, adapted to be arranged in a first storage position and a second use position. The tubular casing is provided with a narrowing section 21, forming a stop abutment for the distal, non-insertable part of the urinary catheter. A tubular insertion aid 3 is detachably connected to the tubular casing, and arranged in the vicinity of the openable end of the tubular casing. The tubular casing is further provided with a braking element 7 arranged in the vicinity of the openable end, the braking element being arranged inside the tubular casing and allowing temporary fixation of the catheter shaft by compression of the braking element through walls of the tubular casing.

## Description

### Technical field of the invention

The present invention relates to a urinary catheter assembly, and preferably a urinary catheter assembly. The invention is particularly related to hydrophilic catheters, and specifically to hydrophilic urinary catheters.

### Background

The present invention relates to a urinary catheter assembly. Urinary catheters are commonly used for draining urine from the bladder. Urinary catheters can be of an indwelling type, for long term use, such as days or even weeks, or for intermittent use, whereby the catheters are used for a single draining procedure, typically lasting a few minutes. Intermittent urinary catheters are e.g. used by a large group of persons for self-catheterization, which is a daily-life procedure, taking place several times a day. Typically, catheters for intermittent catheterization are used by patients suffering from urinary retention, due to e. g. spinal cord injury, Multiple Sclerosis or Prosthatic Hyperplasia. Using an intermittent catheter, the bladder may be drained through a natural or artificial urinary canal. Many catheters, such as those for intermittent catheterization, are provided with a hydrophilic coating or the like, providing a smooth and slippery surface when wetted, for safe and comfortable insertion in the urinary canal.

Individuals who suffer from urinary incontinence will normally self-catheterize several times a day. Self-catheterization involves removing the catheter assembly from its package and inserting and advancing the catheter tube through the user's urethra. However, preparation and manipulation of known catheter assemblies is often complicated and tedious. Further, often users of intermittent urinary catheters have limited or diminished dexterity, e.g. as a result of spinal cord injuries. Also, users of intermittent catheters are often required to self-catheterize outside the privacy of the home, such as in public restrooms. Thus, for these and other reasons, it is desirable that the intermittent catheters are provided in discrete packaging that is easy to open and manipulate, which are compact and portable, and wherein the catheter can be deployed and used in a way that alleviates concerns about inadvertent urine leakage or spillage and avoids pain or discomfort to the user.

In particular, male catheters are relatively long, making the catheter assemblies large and difficult to handle.

Thus, there is still a need for improved urinary catheter assemblies, and in particular male urinary catheter assemblies. The assembly should preferably be relatively simple and cost-efficient to produce. Further, the assembly should be easy and intuitive to open and use, even for users with reduced dexterity. The assembly should also preferably be rather small, so that it can easily be carried around by the user in his/her daily life. There is further a need to facilitate drainage of urine into appropriate places, such as into a toilet, and in particular without the need for being very close to the toilet. Thus, there is a general need for a more simplified catheterization procedure.

### Summary of the invention

It is therefore an object of the present invention to provide a urinary catheter assembly which at least alleviates the above-discussed problems.

This object is obtained by means of a urinary catheter assembly in accordance with the appended claims.

According to a first aspect of the present invention there is provided a urinary catheter assembly comprising:
a urinary catheter having a proximal part with an insertion tip, a distal part and a tubular shaft extending there between, wherein the distal part, on a least a part thereof, has a greater cross-sectional dimension than the tubular shaft and the proximal part;
a tubular casing being adapted to be arranged in a first storage position and a second use position and with an openable distal end, wherein in the first storage position, the tubular casing is arranged to accommodate at least the insertable part of the urinary catheter, in which the tubular casing forms a sealed and closed compartment around the insertable part of the urinary catheter, and wherein in the second use position, the openable end of the tubular casing is opened, allowing the insertable part of the urinary catheter to be expelled out from the tubular casing, wherein the tubular casing, in a proximal part thereof, is provided with a narrowing section, providing a decreased internal diameter compared to at least an adjacent, more distal part of the tubular casing, the narrowing section forming a stop abutment for the distal part of the urinary catheter; and
a tubular insertion aid being detachable connected to the tubular casing, and arranged in the vicinity of the openable end of the tubular casing;
wherein the tubular casing is further provided with a braking element arranged in the vicinity of the openable end, the braking element being arranged inside the tubular casing and allowing temporary fixation of the catheter shaft by compression of the braking element through walls of the tubular casing.

It has been found by the present inventors that a braking element, enabling simple and intuitive temporary fixation of the catheter in relation to the package, is highly advantageous for this type of urinary catheter assemblies, and in particular when used in conjunction with a tubular insertion aid. The brake makes it simple to control the catheter shaft during movement and repositioning of the insertion aid, thereby improving and facilitating the overall catheterization process. Further details of this is discussed in the following.

The braking element preferably comprises at least one braking arm extending in parallel with the catheter shaft, and with a free end. In one embodiment, the at least one braking arm comprises a plurality of braking arms, such as two, three or four arms, or even more arms, the arms being separately arranged around the circumference of the catheter shaft. Preferably, the arms are arranged equidistantly from each other in a circumferential direction.

The braking arms may be provided with an inward sharp protrusion facing the catheter shaft, and being arranged at or in the vicinity of the free end.

The tubular insertion aid may have a circular cross-section, but alternative shapes, such as rectangular and semi-circular shapes are also feasible. Further, the tubular insertion aid may comprise one or several slit openings extending along all or part of the length of the tubular insertion aid.

The tubular insertion aid may be arranged exteriorly on the tubular casing.

In the first storage position, the tubular insertion aid may be arranged overlying the braking element.

The tubular insertion aid may further have a funnel shape, whereby the proximal end has a smaller cross-sectional area than the distal end.

The tubular casing may comprise two open ends being connected together for closure, thereby forming an annular, closed cavity within the tubular casing in the storage position. However, the tubular casing may also be non-annular, and extending along a straight or curved line between two ends. The two ends may both be openable, and may e.g. each comprise a detachable cap.

The urinary catheter assembly may further comprise a drip stop valve arranged at or in the vicinity of the distal part of the catheter, the drip stop valve being arranged to allow liquid to flow through the drip stop valve in a direction from the proximal part to the distal part when the fluid pressure exceeds a threshold value, and to stop flow in said direction when the fluid pressure does not exceed said threshold value.

As will be discussed in greater detail in the following, such a drip stop valve is highly advantageous, since it hinders dripping from the catheter after use, when the catheter e.g. is to be re-inserted into the package for disposal, or to be disposed immediately. In particular, such a drip stop valve has great advantages in conjunction with embodiments where the catheter is also useable as an extension tube for the catheter, and in particular an extendible extension tube, since in such embodiments, the rear end of the catheter is generally inaccessible for the user, making other ways of stopping dripping problematic or even impossible.

The tubular casing may be extendible between a relatively shorter length in the first storage position, and a relatively longer length in the second use position.

The urinary catheter assembly may further comprise at least one cap arranged to close one or several openable ends of the tubular casing in the first storage position and to be openable by an opening action. Further, the urinary catheter assembly may comprise a one-way locking element arranged in the vicinity of the openable end of the tubular casing, and arranged to be brought into a one-way locked disposition to lock the catheter shaft for movement in one direction in relation to a proximal part of the tubular casing when said cap is opened by said opening action, said one-way locking disposition preventing displacement of the urinary catheter away from the proximal end of the tubular casing in a first direction towards the distal end but allowing displacement of the urinary catheter in an opposite second direction, and further arranged automatically to release the one-way locking disposition upon movement of the catheter in the second direction.

Hereby, it can easily and efficiently be hindered that the catheter is pulled into the package when it is extended and prolonged. At the same time, the one-way locking element provides no obstacle or hinder for any other, normal action of the catheter, since it is only activated once, immediately following opening of the tubular casing, and immediately is released as the catheter is moved forward.

The catheter may be a hydrophilic catheter, and wherein the assembly further comprises a wetting fluid for activation of the hydrophilic catheter.

The distal part of the catheter may comprise a flared connector.

The casing may, in the first closed position, provide a sterile and moisture proof compartment for the insertable section of the catheter in the closed storage position.

In the present application, the term "proximal" is used to indicate the end or portion of a catheter that is inserted into the body of the user, i.e. the end or portion of the catheter that during use is closer in proximity to the user's body and/or initially enters the user's body upon insertion. The term "distal" is used to refer to an end or portion of the catheter that is opposite the proximal end or portion and is typically further away from the user's body. For the sake of consistency, when the terms "distal" and "proximal" are used in the context of other components, such as the sleeve and the tubular case, which are not intended for introduction into the user's body. For such other components, "proximal" refers to the end or portion that is closer to the proximal end of the catheter when the catheter is housed within the assembly, while "distal" refers to an end or portion located opposite to such proximal end or portion.

In accordance with the present invention, the tubular casing/package serves two purposes. It functions as a package, for maintaining at least the insertable part of the catheter in a clean and sterile condition during storage, prior to use. When the catheter is a hydrophilic catheter, the casing may also accommodate a wetting fluid for activation of the hydrophilic catheter, and preferably for maintaining the catheter continuously wetted and activated during storage. The package can also be resealable, allowing the catheter to be reinserted into the package after use, and preferably to be closable once the catheter has been reinserted. However, the casing also functions as an extension tube, providing a prolongation of the fluid path from the drainage openings at the insertion end of the catheter to a discharge end. Hereby, the drained urine can be discharged into e.g. a toilet even when the user is at some distance from the toilet.

Further, since the package is extendible, it may still present a compact and neat storage position. In this position, the catheter assembly could easily be carried around discretely, e.g. in a handbag, in a pocket or the like, without attracting notice. Further, the catheter assembly can easily be transformed into the use position, in which the package is extended for use as a catheter extension, as discussed in the foregoing. After use, the catheter assembly may be directly discarded. However, it may alternatively be returned to the closed position, so that the used catheter assembly can then be carried by the user in the same discrete way as the unopened assembly, and without the risk of spillage etc, to be discarded later.

The catheter may e.g. be a urinary catheter. The catheter preferably comprises one or several drainage openings, so-called eyes or eyelets, arranged at or in the vicinity of the proximal insertion end. A part of, or the whole, catheter shaft may form an insertable part or insertable length of the catheter. At least the insertable part may further be provided with a hydrophilic coating, or in other ways been provided with a hydrophilic surface, which exhibits a lowered friction when wetted. Further, the distal part may, at least on a part thereof, have larger cross-sectional dimensions than the catheter shaft. The distal part may e.g. be flared or funnel shaped, increasing in dimension towards the distal end, thereby enabling connection of a tube, collection bag or the like.

The entire catheter may be accommodated in the casing, or in a compartment formed by the casing and a cap. However, it is also possible to have a non-insertable part of the catheter arranged outside the casing, so that this exposed part of the catheter, together with a lid or the like, together forms a closure of the tubular casing.

The catheter may be a hydrophilic catheter, having a hydrophilic surface. The hydrophilic surface may be arranged as a hydrophilic coating arranged on a substrate of the catheter, as is per se well known in the art. However, the hydrophilic surface may alternatively be arranged as an integrated part of the catheter, such as an integrated layer, or alternatively, the entire catheter, or part(s) of the catheter, may be made of a hydrophilic material. The hydrophilic surface is preferably arranged to provide low friction when wetted.

In an embodiment of the invention the catheter shaft is arranged to be inserted into a body cavity or body passageway, and the shaft presents the hydrophilic surface on an exterior surface thereof. The hydrophilic surface may be a surface provided with a hydrophilic coating, for example made in accordance with EP 0 093 093 and EP 0 217 771.

The catheter assembly may further comprise a wetting fluid, such as a wetting liquid, for wetting and activation of the hydrophilic surface. The wetting fluid may be arranged in a separate compartment within the assembly, to be released into the compartment housing the catheter shaft at a suitable time, such as immediately prior to use. However, the wetting fluid may also be provided within the same compartment as the catheter shaft, thereby maintaining the catheter in a wetted, activated state also during storage. This wetted state may be present immediately following closing and sealing of the package, or be obtained after some time of storage. The activated state may thus be provided by pre-wetting of the catheter, prior to arrangement of the catheter in the package, but may alternatively be provided after placement of the catheter in the package. Specifically, wetting of the hydrophilic surface may occur during a period of storage in a sealed container by provision of a humid atmosphere in the package, as is per se known in the art, e.g. by being arranged within a hydration element, such as a chamber or sachet which is liquid impermeable and vapor permeable.

Since the hydrophilic surface is then maintained in a wetted state during storage, the medical device is immediately ready-to-use upon removal from the package, and need not be wetted or treated in any way prior to use.

The package/tubular casing is preferably impermeable to the wetting fluid, and preferably made of a gas impermeable material. This ensures that moisture does not penetrate out from the package during storage, and enhances the shelf-life of the product.

The catheter is preferably a urinary catheter, and most preferably a urinary catheter for intermittent, short time use. The term "short term use" indicates a use that is limited in time, and in particular limited to a time period of less than 15 minutes, and preferably less than 10 minutes, and most preferably less than 5 minutes.

The wetting liquid is preferably an aqueous liquid, comprising at least 75% of weight of water, and preferably at least 80% of weight, and more preferably at least 85% of weight, and most preferably at least 90% of weight. In some embodiments, the wetting liquid may be plain water. However, the wetting liquid may also comprise one or more additives, such as an anti-bacterial agent, a pharmaceutical active substance, or the like.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Fig. 1 is a cross-sectional view of a urinary catheter assembly in accordance with one embodiment of the present invention;
Fig. 2 is a side view of the urinary catheter assembly of Fig. 1;
Fig. 3 is a cross-sectional view of a urinary catheter assembly in accordance with another embodiment of the present invention;
Fig. 4 is a cross-sectional and more detailed view of a seal and stop arrangement between the catheter and the package;
Fig. 5 is a side view of a urinary catheter assembly in accordance with another embodiment of the present invention;
Fig. 6a-6c are cross-sectional illustrations of a one-way lock arrangement in accordance with an embodiment of the present invention, wherein Fig. 6a illustrates the one-way lock in an unlocked state, Fig. 6b illustrates the one-way lock during locking, and Fig. 6c illustrates the one-way lock in a locked state;
Fig. 7 is a perspective view of a rear end of a catheter provided with a drip stop valve in accordance with an embodiment of the invention;
Fig. 8 is a perspective view of a catheter provided with a drip stop valve in accordance with another embodiment of the invention;
Fig. 9 illustrate the part of Fig. 1 with the insertion aid in greater detail; and
Fig. 10 is a schematic, cross-sectional view of an embodiment of the braking elements in more detail.

### Detailed description of preferred embodiments

In the following detailed description preferred embodiments of the invention embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the medical device, etc.

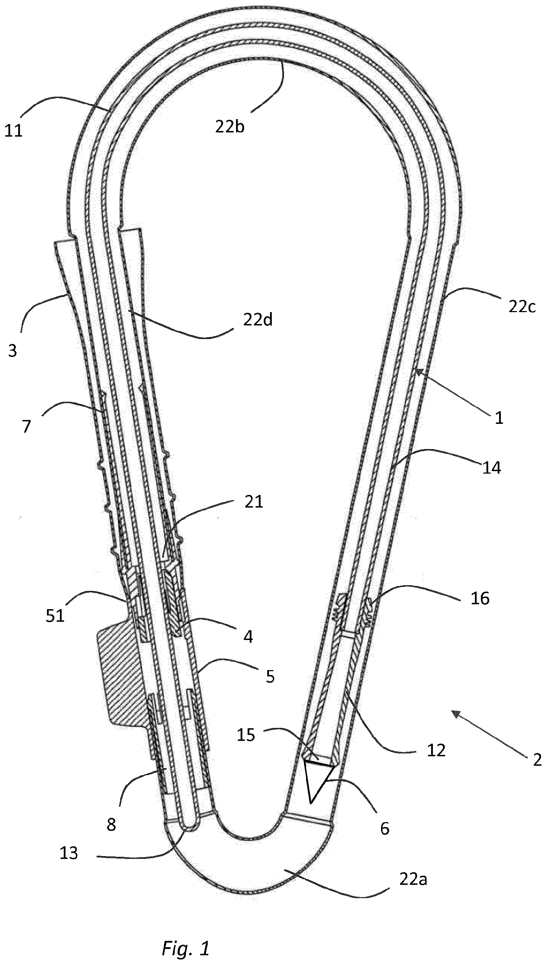

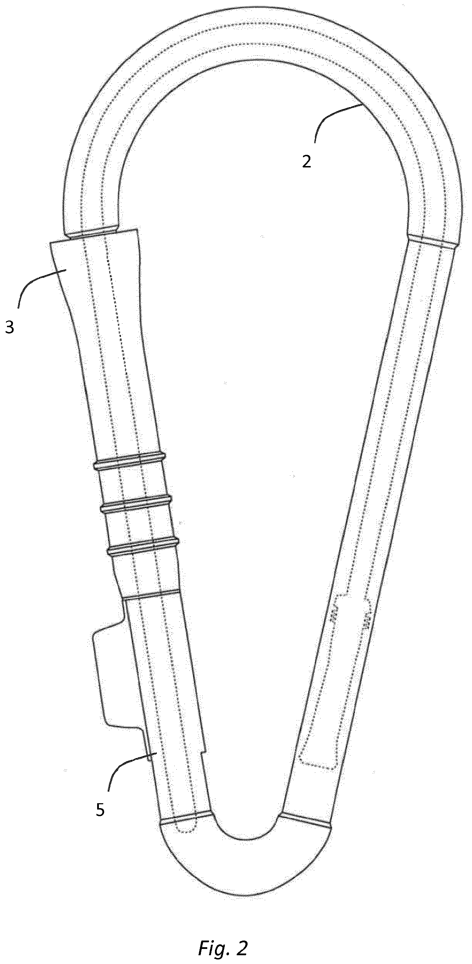

A urinary catheter assembly as illustrated in Figs. 1 and 2 comprises a catheter 1 having an insertable section 11, comprising an insertable, proximal part, with an insertion tip, and a catheter shaft, and a non-insertable section 12, a distal part, forming a connector part. The non-insertable section 12 preferably has a larger diameter than the insertable section 11 at least on a part thereof. The rear end of the non-insertable section may be flared or funnel-shaped, and may be arranged to be connected to a tapered connection part of a urine collection bag or the like. However, the non-insertable section may alternatively have a relatively uniform cross-sectional area.

The catheter is accommodated in a tubular casing, package 2, forming a closed cavity in which the catheter is arranged. The cavity is preferably sterile and moisture proof. To this end, the package may e.g. be made of a fluid impermeable material. The package is preferably, at least in most parts, made of a relatively flexible material. The package may be transparent, but may alternatively be opaque or semi-opaque.

The package is preferably in the form of a tube or hose, and in a preferred embodiment, the package is arranged to form an annular shape, with the cavity extending as an annular cavity inside the package. The annular package may be provided with a breakable or openable member, allowing two ends of the annular package to be separated from each other, for opening of the package upon use. The annular cavity may be closed in the opening area, forming a generally C-shaped annular cavity. However, the annular cavity may also be open also in the opening area, forming a generally O-shaped annular cavity. The annular package embodiment will be discussed in further detail below.

However, alternatively the package may be non-annular, having a straight disposition, or be arranged in a curved disposition, such as in a U-form shape or the like. In such arrangements, the package is closed in both ends in the storage positions, and is arranged to be openable, for example by closing caps or the like, or by the provision of tear or peel opening features at the ends.

The tubular casing is preferably arranged to narrowly surround the catheter.

In the exemplary embodiment, the tubular casing is useable as an extension of the catheter. Hereby, the enlarged part of the non-insertable section, i.e. the part forming a larger diameter than the insertable section, is primarily arranged to form an engagement with an abutment 21 of the package, when the insertable section has been expelled out from a proximal end of the package, to form a seal with the package, thereby allowing the package to be used as an extension for the catheter. This will be discussed in further detail in the following. To this end, the tubular casing/package is preferably provided with a narrowing section, providing a decreased internal diameter compared to at least an adjacent, more distal part of the tubular casing, the narrowing section forming a stop abutment for the distal part of the urinary catheter.

Thus, in such an embodiment, the package serves two purposes, first to provide a sealed and preferably sterile package for the catheter during storage, and secondly, to serve as an extension tube, for discharging the urine into a toilet bowl or the like.

The package is preferably extendible, thereby allowing the package to have a first length in a first, storage position, and then be extended into a second, longer length a second, use position. Embodiments of such extendible packages will be discussed with greater detail in the following. Such an extendible extension is even more useful than a non-extendible extension, since it may provide a relatively short length and compact assembly in the storage position, and yet provide a long and versatile extension in the use position.

At least a part of the insertable section 11 forms an insertable length to be inserted through a urethra of the user.

The insertable section comprises an insertion tip 13, which may be a closed, rounded end. Further the insertable section may comprise inlet openings (not shown), arranged in the vicinity of the insertion tip, so called catheter eyes or eyelets, leading into a lumen 14 extending through the catheter, and into a discharge outlet 15 arranged at the rearward end of the non-insertable section.

The insertable section may be 80-140 mm for a female user and 200-350 mm for a male user.

The insertable section 11 may comprise a hydrophilic surface, and form a hydrophilic catheter, as is per se well known in the art. The hydrophilic surface may be in the form of a hydrophilic surface coating, for example PVP, and which provides a low-friction surface when wetted with a wetting fluid. Typically, the insertable length is within 80-140 mm for a female patient and 200-350 mm for a male patient. Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. However, instead of a hydrophilic surface coating, the entire insertable section of the catheter may be formed of a hydrophilic material.

In case a hydrophilic catheter is used, the urinary catheter assembly may also comprise a wetting fluid. However, the wetting fluid for activation of the catheter need not be provided within the package. Instead, a wetting fluid may be poured into the package after opening of the package, for wetting of the catheter while it still remains in the package. In some occasions, the catheter may even be removed from the package and wetted e.g. in a different container, even though this is normally not preferred.

However, preferably the wetting fluid is arranged within the assembly, and preferably within the casing, so that the hydrophilic surface of the catheter can be activated even before opening of the package. In one embodiment, the wetting fluid is arranged separated from the catheter, in a wetting fluid container (not shown), such as a pouch or a sachet, or in a separate compartment of the package. The wetting fluid container or compartment is openable by means of e.g. exerting a pressure to the container or compartment, whereby the wetting fluid is released into the package, thereby wetting the hydrophilic surface of the catheter. The wetting fluid container or compartment may also be arranged to open automatically when the catheter assembly is opened, e.g. by the opening of the cap. In another embodiment, the wetting fluid is arranged directly in the compartment of the casing accommodating the catheter, so that the hydrophilic surface of the catheter is in direct contact with the wetting fluid during storage, and thereby is maintained in an activated, ready-to-use state.

The wetting fluid is preferably a liquid, and most preferably an aqueous liquid, such as water or saline. However, the wetting fluid may also be a gas, providing a moist atmosphere in the package sufficient for activation of the hydrophilic surface. Thus, the wetting fluid may be any fluid, gas or liquid, that wets/activates a hydrophilic surface of the catheter.

However, alternatively the catheter may be non-hydrophilic, and may e.g. be lubricated with gel prior to insertion.

In case a non-hydrophilic catheter is used, the urinary catheter assembly may comprise a supply of lubricant, such as a compartment arranged overlying the catheter shaft, so that the shaft is lubricated while being pulled out from the tubular case.

The catheter may further be provided with a drip-stop valve, preferably arranged in or at the non-insertable section 12, and e.g. connected to or integrated with the proximal end of the non-insertable section 12. The drip-stop valve will be discussed in further detail in the following. The drip stop valve may be arranged to allow liquid to flow through the drip stop valve in a direction from the proximal part to the distal part when the fluid pressure exceeds a threshold value, and to stop flow in said direction when the fluid pressure does not exceed said threshold value. The drip stop is of particular advantage for catheter assemblies where the package/tubular casing is used as an extension for the catheter, and in particular for extendible extensions, since in such assemblies, the rear end of the catheter is not directly accessible for the user, which makes it difficult to prevent drips from falling out from the catheter after use. However, with a drip stop valve, drips will be efficiently prevented in an automated fashion.

The urinary catheter assembly may further comprise a tubular insertion aid 3, for use as an aid when manipulating the catheter during or after having expelled the insertable section 11 out from the package for use. The insertion aid will be discussed in more detail in the following. The tubular insertion aid is preferably detachably connected to the tubular casing, and arranged in the vicinity of the openable end of the tubular casing.

The urinary catheter assembly may further comprise a braking element 7 arranged in the vicinity of the openable end, the braking element 7 being arranged inside the tubular casing and allowing temporary fixation of the catheter shaft by compression of the braking element through walls of the tubular casing. The braking element is of particular advantage in combination with a tubular insertion aid, since it allows a simple and effective way of retaining the catheter during movement and repositioning of the tubular insertion aid. Embodiments of the braking element will be discussed in further detail below.

The urinary catheter assembly may also comprise a cap arranged to close the openable end of the tubular casing in the first storage position, and to be openable by an opening action. In case the package is an annular tubular casing, the cap may be arranged as a part of the annular tubular casing, and be arranged to break-up the annular cavity at will, when the catheter is to be used.

In one embodiment, the urinary catheter assembly further comprises a one-way locking element arranged in the vicinity of the openable end of the tubular casing, and arranged to be brought into a one-way locked disposition to lock the catheter shaft for movement in one direction in relation to a proximal part of the tubular casing when said cap is opened by the opening action. The one-way locking disposition preferably prevents displacement of the urinary catheter away from the proximal end of the tubular casing in a first direction towards the distal end but allows displacement of the urinary catheter in an opposite second direction. Further, the one-way locking disposition may be arranged automatically to be released upon movement of the catheter in the second direction. Such a one-way lock may be used in combination with any of the above-discussed embodiments, but is of particular advantage in combination with embodiments comprising a package/tubular casing forming an extendible extension. Due to the one-way lock, the catheter is locked to a proximal part of the package immediately upon opening. This prevents that the catheter is pulled away from the opening, i.e. backwards, into the package, when the ends of the tubular casing are pulled apart to extend the package. Thus, the catheter remains in place in relation to the proximal end of the package even when the package is extended. Thereafter, when the catheter is to be expelled from the package, the one-way lock allows forward movement, i.e. out of the package/tubular casing, and also immediately becomes released upon such movement. This is of advantage, since it allows the catheter to be repositioned into the package/tubular casing after use. Once the catheter is reinserted into the tubular casing and reclosed, the package will retain all or at least some of its original sealing qualities, and will consequently not leak and protect the user from spillage and contamination. Possible realizations of such a one-way lock will be discussed in more detail in the following.

The specific features of the urinary catheter assembly, to be discussed in more detail below, are preferably provided in combination. However, these features may also be used separately, or in any combinations with two, three or more of them.

### Package useable as an extension

The tubular casing, i.e. the package, is preferably useable as an extension of the catheter. Hereby, an enlarged part 16 of the non-insertable section 12, i.e. the part forming a larger diameter than the insertable section, is arranged to form an engagement with an abutment 21 of the package, when the insertable section has been expelled out from a proximal end of the package. Thus, the insertable section of the catheter is extracted out from the package, with the insertion tip leading the way.

As the enlarged part 16 of the non-insertable section 12 reaches the abutment 21, the catheter is arrested, and prevented from moving further out from the package. At the same time, a seal is formed between the package and the catheter.

In this state, the lumen of the package is in fluid communication, via the non-insertable section, into the package, and through the package to the distal end, through which urine may then be discharged. Thus, in this state, the package serves as an external extension of the catheter.

The abutment 21 is arranged as a narrowing section, providing a decreased internal diameter compared to at least an adjacent, more distal part of the tubular casing. The decreased internal diameter of the abutment 21 is at least as great as the external diameter of the insertable section 11 of the catheter, and preferably at least somewhat larger. At the same time the internal diameter of the abutment 21 is smaller, and preferably significantly smaller than the external diameter of the enlarged part 16 of the catheter.

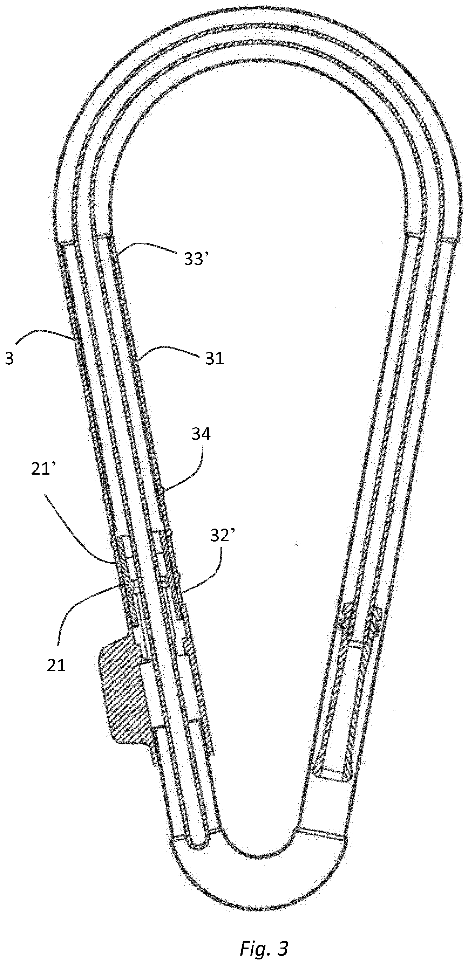

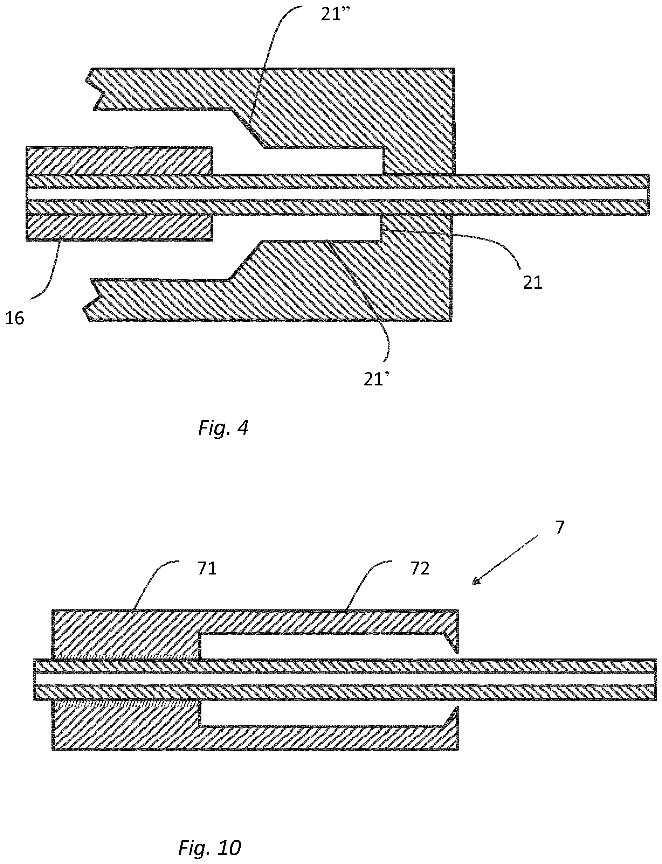

The seal and stop may be formed only at one distinct position, as in the embodiment illustrated in Fig. 1. However, in order to be more versatile, and also to provide a tighter seal, a transition area may also be formed during which a seal is formed, but where the movement of the catheter is not stopped. Such an embodiment is schematically illustrated in Fig. 3, and an alternative embodiment is shown with further detail in Fig. 4.

In this embodiment, a transition area 21' is provided between the abutment 21 and the rest of the package 2. The abutment 21 is essentially arranged in the same way as in the previously discussed embodiment. In the transition area 21', the internal diameter is slightly larger than the internal diameter at the abutment 21, and slightly smaller than the internal diameter in the adjacent part of the rest of the package. The internal diameter of the transition area 21' is about equal to the external diameter of the enlarged part 16 of the catheter. Hereby, the enlarged part 16 and the transition area 21' may form a sliding seal, preventing liquid to escape, but still allowing movement of the catheter in a longitudinal direction. At the distal end of the transition area 21', a tapering section 21" may be provided, to ensure that the enlarged part 16 is centered and finds its way into the transition area 21' as the catheter is expelled out from the package.

Thus, in this embodiment, a seal is formed immediately as the enlarged part reaches the transition area 21', but the catheter is not stopped from further longitudinal movement until it reaches the abutment 21. Hereby, a more versatile functionality of the product is obtained, enabling adequate sealing properties for several extraction positions, and also providing an improved seal at the end position, at the abutment 21.

The transition area 21' may be very limited, reaching over only a few mm or cm, such as 1-50 mm, and preferably in the range 5-30 mm, and most preferably in the range 10-25 mm. However, longer transition areas 21' may also be used, such as having a length of up to 10 cm, or even longer.

When the package also enables use as an extension, the package serves two purposes: first to provide a sealed and preferably sterile package for the catheter during storage, and secondly, to serve as an extension tube, for discharging the urine into a toilet bowl or the like.

### Extendible extension package

For packages useable as catheter extensions, it is of advantage if the packages are also extendible. Such an extendible package allows the package to have a first length in a first, storage position, and then be extended into a second, longer length a second, use position. Such an extendible extension is even more useful than a non-extendible extension, since it may provide a relatively short length and compact assembly in the storage position, and yet provide a long and versatile extension in the use position.

The extendible package may be extendible by pulling the ends of the tubular casing apart. The tubular casing may comprise one or several sections which are extendible, or even be extendible over its entire length.

The extendible section(s) may comprise a tubular wall that are formed as a folded wall, a corrugated wall or the like. However, additionally or alternatively, the tubular wall may comprise a non-elastically stretchable wall.

The advantage of this embodiment is that the catheter assembly in its stored condition takes up very little room and is therefore handy to carry along. When the catheterization takes place, the catheter package acts as an extension for the catheter in order to drain the urine flow through the catheter directly into a toilet or other available collection or drainage means without spillage and leaving only the catheter and its package as items which must be disposed after use. The catheter may also be reassembled within the package after use, for later disposal.

The extendible section(s) are preferably arranged to be maintained in the extended position after having been brought to this state. The extension may be reversible or irreversible, i.e. the tubular casing can allow the package to be brought into the extended position, and then back to the compact storage length, or alternatively only allowing the package to be brought from a compact state to an extended state. In a preferred embodiment, at least two extendible sections are provided, separated by at least two non-extendible sections.

Each extendible section is preferably arranged to be extendible in such a way that its length in the extended state is at least 25% longer than the length of the same section in the compact state, and preferably at least 50% longer, and more preferably at least 75% longer, and most preferably at least 100% longer.

In the compact storage position, the package may have a length essentially of the same length as the catheter itself, or somewhat longer. In the extended use position, the length may be significantly longer, such as having twice the length of the catheter. The extension can also, preferably, be controlled to various length between the compact state and the fully extended state, allowing the user to control how long the extension should be between these states, depending on e.g. the particular circumstances at hand, and the desire of the user.

Preferably, longer maximal extendibility may be provided for female catheter assemblies than for male catheter assemblies. For female catheter assemblies, an extendibility of 100-200% may e.g. be provided, or even longer, and for male catheter assemblies, an extendibility of 50-100% would often suffice.

In the embodiment of Figs. 1 and 2, sections 22a and 22b are arranged as extendible sections, and e.g. comprising non-elastically stretchable material. Thus, the extendible sections can here be irreversibly extended and prolonged by pulling the ends of the package apart.

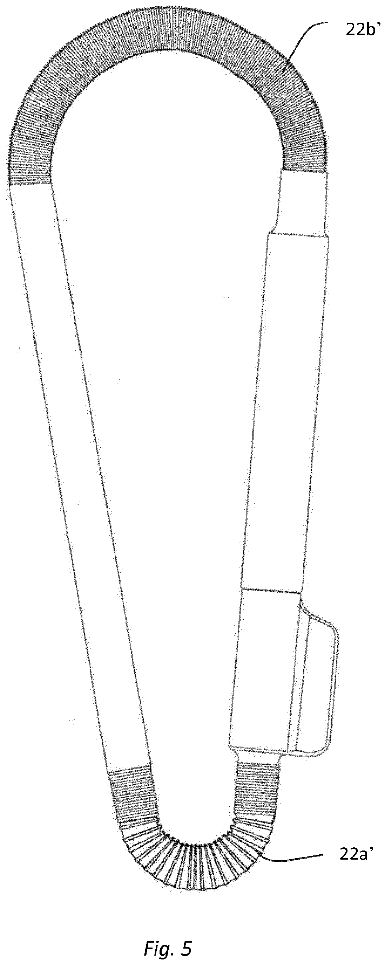

In the embodiment of Fig. 5, the sections 22a' and 22b' are provided with accordion-like corrugations, of a similar type as is used in bendable straws. These sections are also extendible by pulling the ends of the package apart, and this action may also be reversed, by pushing the package ends towards each other, to again compact the package after use.

The corrugations may have relatively sharp edges. The corrugated section may be bent without essentially narrowing the opening thereof, and may be elongated simply by stretching the corrugations, and may be shortened by simply pushing the ends together re-establishing the corrugations.

When the tubular catheter package with the corrugations is elongated the corrugations will enable the catheter package to stay in the extended position given by the user during the catheterization and until the user changes the position. For example, the user may, after the catheterization, push the catheter package back to its initial position by pressing the corrugations together. Such handling is very easy to perform even for users with poor dexterity.

The extendible sections are also well suited to maintain a defined curvature of the package. Thus, in the storage position, the extendible sections may be arranged to maintain a defined radius in the curved sections of the package, thereby avoiding kinking and deformation of the catheter during storage.

The corrugated section may comprise pleats, and the pleats may form a bellow-like section of the tubular casing.

### One-way locking arrangement

Referring to Figs. 1 and 2, the urinary catheter assembly may also comprise a cap 5 arranged to close the openable end of the tubular casing in the first storage position, and to be openable by an opening action. In case the package is an annular tubular casing, as in the embodiments of Figs. 1 and 2, the cap 5 may be arranged as a part of the annular tubular casing 2, and be arranged to break-up or open the annular cavity at will, when the catheter is to be used. For example, the cap may be arranged as a sleeve arranged over the distal end of the tubular casing, and fixedly connected thereto. At the other end, the sleeve is separably connected to the proximal end of the tubular casing by a joint 51. The joint may be in the form a friction fit, a breakable weld, or the like. However, preferably the joint is a screw joint, allowing the cap 5 to be opened up by twisting/rotating the cap in relation to the package 2. To this end, the proximal part of the package may e.g. be provided with external threads, and the cap may be provided with corresponding internal threads.

In one embodiment, the urinary catheter assembly further comprises a one-way locking element 4, as seen in Fig. 1, arranged in the vicinity of the openable end of the tubular casing, and arranged to be brought into a one-way locked disposition to lock the catheter shaft for movement in one direction in relation to a proximal part of the tubular casing when said cap is opened by the opening action. The one-way locking disposition preferably prevents displacement of the urinary catheter away from the proximal end of the tubular casing in a first direction towards the distal end but allows displacement of the urinary catheter in an opposite second direction. Further, the one-way locking disposition may be arranged automatically to be released upon movement of the catheter in the second direction.

Such a one-way lock may be used in combination with any of the above-discussed embodiments, but is of particular advantage in combination with embodiments comprising a package/tubular casing forming an extendible extension.

Due to the one-way lock, the catheter is locked to a proximal part of the package immediately upon opening. This prevents that the catheter is pulled away from the opening, i.e. is retracted backwards, into the package/tubular casing, when the ends of the tubular casing are pulled apart to extend the package. Thus, the catheter remains in place in relation to the proximal end of the package even when the package is extended. Thereafter, when the catheter is to be expelled from the package, the one-way lock allows forward movement, and also immediately becomes released upon such movement. This is of advantage, since it allows the catheter to be repositioned into the package/tubular casing after use. Once the catheter is reinserted into the tubular casing and reclosed, the package will retain all or at least some of its original sealing qualities, and will consequently not leak and protect the user from spillage and contamination.

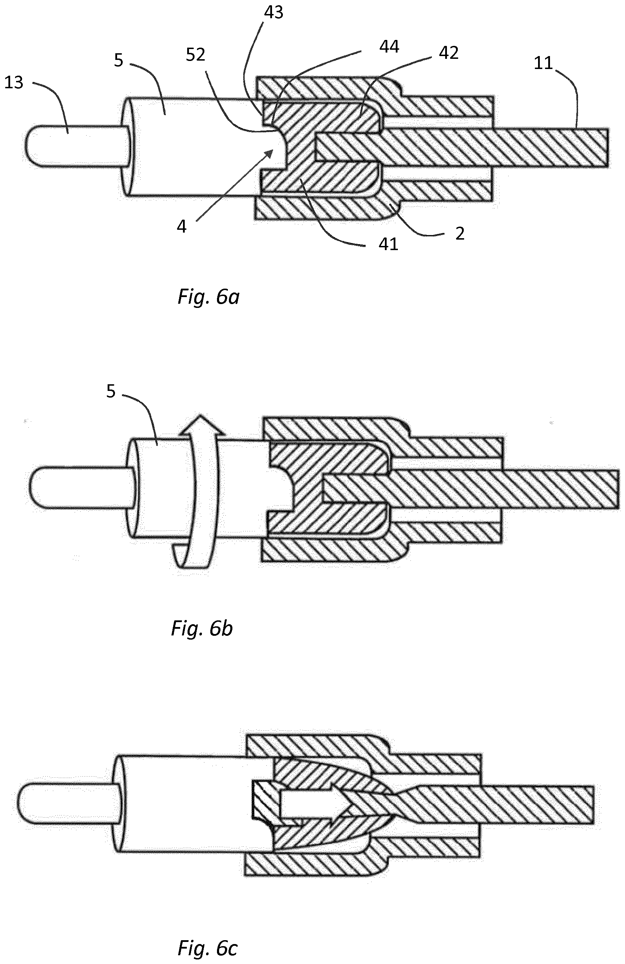

In one embodiment, illustrated in Figs. 6a-6c, the one-way lock 4 comprises a tubular sleeve 41 of an elastically compressible material, arranged around the proximal part of the insertable section 11 of the catheter, and with the insertion tip 13 preferably being arranged outside the sleeve. The sleeve has a distal end 42 facing a narrowing restriction formed in the proximal part of the tubular casing 2, and an opposite, proximal end 43 facing the cap 5.

The distal end 42 facing the narrow restriction may be provided with a solid circumference, and may have the same material thickness as the rest of the sleeve. However, preferably, the material thickness is lower towards the distal end. For example, the distal end of the sleeve may comprise longitudinally directed fingers extending in the distal direction, as in the illustrative example. Alternatively, or additionally, the distal end of the sleeve may have a tapering thickness in the distal direction.

The proximal end 43 facing the cap 5 is preferably provided with curved rim, forming slanted areas 44. The slanted areas may e.g. be arranged as depressions or cut-outs with curved side walls. The cap is provided with protrusions 52, preferably provided with a correspondingly slanted surfaces, to engage with the slanted areas 44 of the sleeve.

Preferably, the sleeve comprises two, three or more slanted areas 44, evenly distributed around the circumference of the sleeve, and preferably also a corresponding number of protrusions 52.

The sleeve 41 is loosely held within the cavity formed around it by the proximal part of the tubular casing 2 and the cap 5, but due to the friction it preferably restricts rotation.

Upon opening of the cap 5, the cap 5 may be twisted or rotated in relation to the tubular casing 2, as discussed in the foregoing. Upon twisting/rotation of the cap, as illustrated in Fig. 6b, the protrusion(s) 52 will climb up on the slanted area(s) 44, thereby forcing the sleeve downwards, away from the cap. Such downward movement forces the distal end of the sleeve to be wedged in between the narrow restriction of the package and the catheter shaft, as shown in Fig. 6c, thereby effectively locking the catheter shaft and the proximal part of the package to each other.

Thus, the locking of the catheter to the proximal part of the package is obtained automatically, as a consequence of performing the normal opening action of the package, i.e. the twisting of the cap for opening of the package. The catheter then remains locked to the package, and e.g. hinders downward movement of the catheter into the package if the package ends are pulled apart for extension.

However, as soon as the catheter is moved forward, e.g. in order to be expelled for use, the wedged in distal end of the sleeve is released from its locked position, and the catheter can be moved out in the proximal direction. Further, after release of the sleeve from its wedged in locked position, and after removal of the cap, the sleeve will also not hinder movement of the catheter in the distal direction, thereby e.g. allowing the catheter to be re-inserted into the package after use.

### Drip stop valve

The catheter may further be provided with a drip-stop valve, preferably arranged in or at the non-insertable section 12, and e.g. connected to or integrated with the distal end of the non-insertable section 12. The drip stop valve may be arranged to allow liquid to flow through the drip stop valve in a direction from the proximal part to the distal part when the fluid pressure exceeds a threshold value, and to stop flow in said direction when the fluid pressure does not exceed said threshold value.

The drip-stop valve may also function as a one-way valve, preventing flow in an opposite direction, i.e. in the direction from the distal part to the proximal part. However, alternatively, the drip-stop valve may allow flow in such an opposite direction, since flow in this direction will normally anyway never occur during catheterization.

The drip-stop valve prevents that urine drips from the rearward end of the catheter after drainage of the urine, and during the subsequent handling of the catheter. This is of general advantage for most types of urinary catheter assemblies. However, in many known catheter assemblies, the rearward end of the catheter is exposed and accessible during catheterization, allowing the user to close the end after catheterization, e.g. by putting a finger over the opening. However, in other catheter assemblies, such as in the ones discussed above, the rearward end of the catheter remains inaccessible during catheterization, e.g. by being arranged within a package also serving as an extension tube. The drip-stop valve is of particular advantage for such catheter assemblies, such as catheter assemblies where the package/tubular casing is used as an extension for the catheter, and in particular for extendible extensions. With a drip stop valve, dripping will be efficiently prevented in an automated fashion.

The threshold value may be set to a very low value, and still enable the intended functionality to be achieved. The threshold value may be set to a value preventing urine remaining in the catheter after drainage of the bladder to be discharged through the drip stop valve. Thus, the threshold value may be greater than a pressure obtained from what liquid remaining in the catheter exerts due to gravity. Thus, the threshold value may be set to a value preventing urine remaining in the catheter after drainage of the bladder to be discharged through the drip stop valve. On the other hand, the threshold value is preferably low enough to offer very little resistance to normal discharge of urine from the bladder through the catheter, so that the draining of urine from the bladder is initiated immediately upon insertion of the catheter insertion tip into the bladder, and so that draining of urine from the bladder continuous until the bladder is empty or close to empty.

In one embodiment, the drip stop valve may be provided in the form of a tubular part of a flexible material, said tubular part being connected to the rearward end of the catheter and extending past the rearward end in a direction opposite to the insertion end. The tubular part may comprise two openings, one of which is sealingly connected to the rearward end of the catheter, and the other presenting an open end opposite to said sealingly connected end.

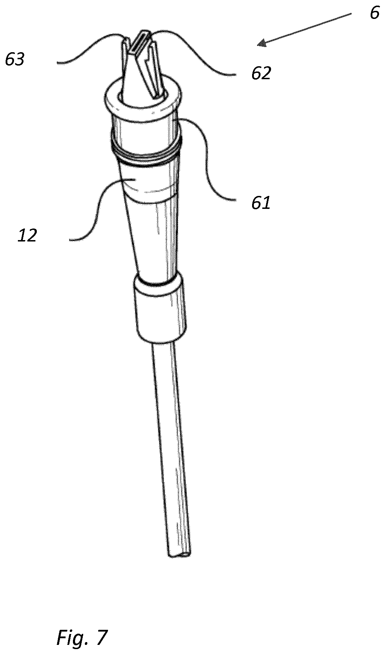

According to one embodiment, the drip-stop valve 6 is provided in the form of a duck bill valve. Such an embodiment is illustrated in Fig. 7. In this embodiment, the tubular part comprises a unit made in one piece, e.g. by molding, and made of a flexible material, such as silicone. The tubular part of this embodiment comprises a cylindrical base part 61, with a diameter essentially corresponding to the end of the rearward part of the catheter. Hereby, the tubular part may be connected to the end surface of the rearward part by means of e.g. gluing or welding. Further, the tubular part comprises an outlet opening part 62 formed in the shape of a duck bill, forming a slit shaped opening at the end. Hereby, a drip-stop valve is formed, since the duck bill end will allow passage of urine coming through the catheter at a certain pressure, above the threshold value, but will prevent passage below said threshold pressure value. Stabilizing tongues 63 may be formed so that they extend outside of the duck bill opening. These stabilizers will allow a more smooth and stable operation of the duck bill, and will also prevent harmonic oscillations from occurring during use, as well as possibly incurred noise caused by such oscillations.

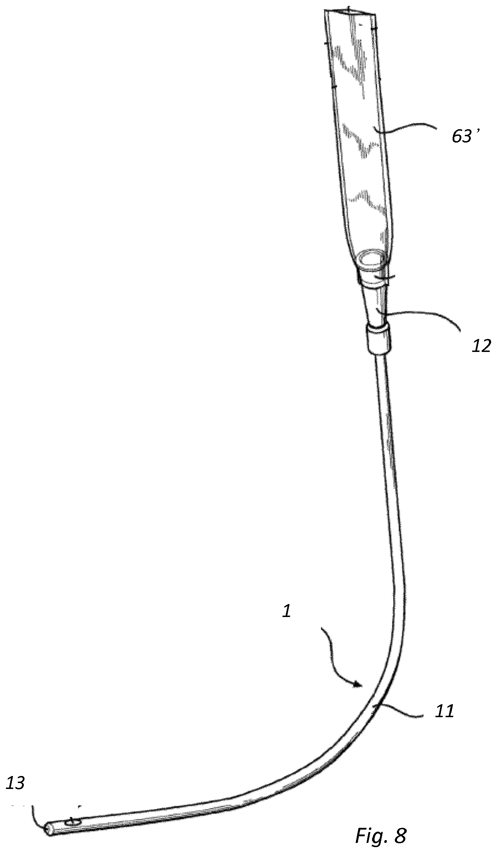

In an alternative embodiment, illustrated in Fig. 8, the drip-stop valve comprises a tubular part 63', such as a hose, of a flexible material, having one end connected to the rearward part of the catheter, and extending past the rearward end in a direction opposite to the insertion end. The other end of the tubular part is freely ending, and provides an outlet for the catheter. The inlet of the tubular parts remains substantially open for passage of fluids there through at all times, whereas the outlet is biased to closure after discharge of fluids through said outlet when the outflow pressure is diminished below a threshold value.

To provide such a suitable threshold value, the tubular part preferably has an elongated form, wherein the length of the tubular part is at least three times the diameter of the rearward end of the catheter, and preferably at least five times said diameter, and most preferably at least ten times said diameter.

The length of the tubular part may be at least 30 mm, and preferably at least 80 mm.

The tubular part/hose may have a perimeter attached along an exterior periphery of the rearward end of the catheter, and may be connected to the rearward part of the catheter by means of at least one of the following methods: shrinkage, force fitting, welding and adhesive bonding.

Further, the tubular part/hose may be funnel shaped, and may e.g. have an inner diameter which is wider at the outlet than at the inlet, wherein the outlet inner diameter preferably is at least twice the inlet inner diameter. Hereby, a gradual or continuous, widening of the lumen through the drip-stop valve may be obtained.

The tubular part/hose is preferably made of a plastic material, and preferably of a relatively thin and flexible material, such as having a wall thickness of less than 0.5 mm, and most preferably less than 0.3 mm.

The tubular part/hose may have a discharge opening directed in the longitudinal direction of the catheter. However, alternatively, the discharge openings may be arranged towards the sides.

### Insertion aid

The urinary catheter assembly may further comprise a tubular insertion aid 3, for use as an aid when manipulating the catheter during or after having expelled the insertable section 11 out from the package for use. The insertion aid will be discussed in more detail in the following. The tubular insertion aid is preferably detachably connected to the tubular casing, and arranged in the vicinity of the openable end of the tubular casing.

The tubular insertion aid may be cylindrical, with a generally uniform diameter along its length. However, it may also have a slightly conical, flared shape, over its entire length or along part(s) of the length.

The tubular insertion aid is preferably made of a relatively soft and flexible material, allowing the catheter to be gripped by applying a moderate finger pressure on the insertion aid. The insertion aid may be provided with a solid wall around its full circumference. However, the insertion aid may also comprise one or several slit openings, extending along the entire length of the insertion aid, or over only part(s) of the length. For example, a slit opening may extend from the distal end of the insertion aid, and towards the proximal end, over a length of e.g. 1/3 or 1/2 of the full length of the insertion aid.

The tubular insertion aid is preferably made of a flexible plastics material. The material may be any thermoplastic and/or thermosetting plastic materials which are usable for providing sufficient strength and flexibility for the intended use. In one embodiment, the tubular insertion aid may be made of thermoplastic elastomer, such as the commercially available Dryflex(R).

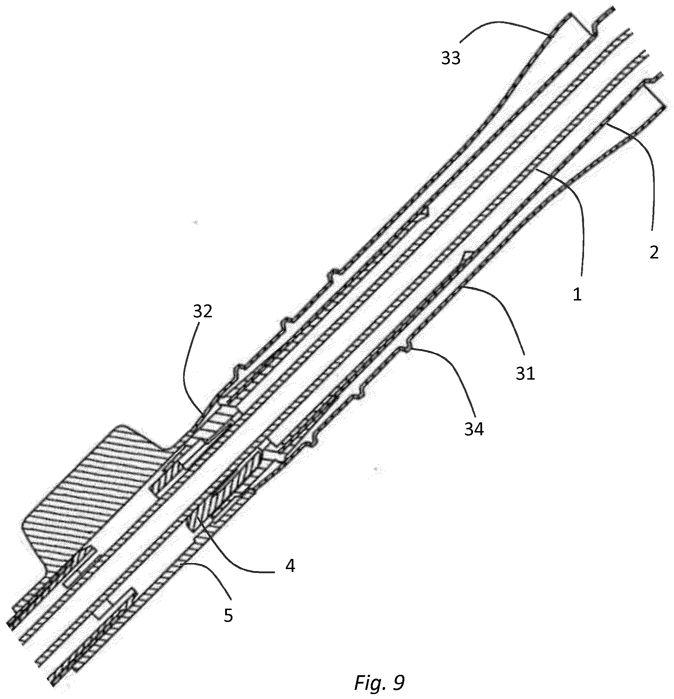

In one embodiment, as illustrated in Fig. 1, and in further detail in Fig. 9, the tubular insertion 3 comprises a solid wall around its full circumference. The insertion aid comprises a generally cylindrical central part 31, an inwardly conical, tapering proximal part 32, including the proximal end, and an outwardly flared, funnel shaped distal part 33, including the distal end. Hereby, the diameter at the forward, proximal end is smaller than the diameter at the central part, and with a gradual, smooth transition there between, and the diameter at the rearward, distal end is larger than the diameter at the central part, and with a gradual, smooth transition there between.

In this embodiment, the tubular insertion aid is arranged exteriorly around a proximal part of the tubular casing, i.e. the package. The internal diameter of the distal part 33, and in particular the distal end of the insertion aid, is preferably greater than the external diameter of the package at a corresponding position, i.e. where the insertion aid overlies the package. The internal diameter of the central part 31 is preferably also greater than the external diameter of the package. Hereby, the insertion aid becomes more loosely arranged on the package, and is easier to remove for use. However, alternatively, the insertion aid may, at the central part, have an internal diameter that is only slightly larger than the corresponding external diameter of the package, or even an internal diameter that is about the same as the external diameter. The proximal part 31, and in particular the proximal end of the insertion aid, preferably has a diameter about equal to the external diameter of the corresponding part of the package.

Thus, the tubular insertion aid is preferably at least partly funnel shaped. In a preferred embodiment, a portion of the insertion aid is at least partly funnel shaped, whereas another portion has an essentially uniform diameter. It is also preferred that the rearward, distal opening has a greater cross-sectional dimension than the forward, proximal opening.

The proximal part 31, and in particular the proximal end, is preferably connected and fixated to the proximal part of the package, thereby maintaining the insertion aid in this fixed position until it at will is separated from the package, for use as an insertion aid. The connection can be realized in various ways, such as by friction fit, welding, adhesion, etc.

Additionally, or alternatively, the proximal part, and in particular the proximal end, of the insertion aid may be connected to the cap 5. This connection may e.g. be arranged so that the insertion aid is disconnected automatically upon opening of the package, e.g. by twisting or rotating the cap 5 into an open state.

In order to facilitate removal of the insertion aid, it may further be provided with a surface texture or the like on at least part of its exterior surface, to provide improved gripping properties. In the illustrative example, the insertion aid is provided with corrugations 34 extending around the circumference of the insertion aid. This makes gripping and squeezing of the insertion aid easier, which facilitates detachment of the tubular insertion aid from the package, and the use of the insertion aid for maneuvering the catheter. The gripping means may alternatively be realized in other ways, such as by at least one of outward protrusions, embossment and perforations.

In an alternative embodiment of the insertion aid, as illustrated in Fig. 3, the insertion aid has an overall generally cylindrical shape, with about the same diameter over its entire length. Thus, here the proximal part 32' has about the same diameter as the central part 31, and the distal part 33' may also have about the same diameter as the central part. The internal diameter of these parts may all be about the same as, or only slightly larger than, the corresponding external diameter of the package. In this exemplary embodiment, the connection between the insertion aid and the package may be provided at any of the proximal, central or distal parts, or at two or more of the parts, or even over the entire length of the tubular insertion aid.

The insertion aid is preferably arranged overlying the braking element 7, to be discussed in more detail in the following.

The insertion aid may be used for catheter manipulation, thereby avoiding the need to directly touch the catheter, and in particular the insertable part of the catheter. This reduces the risk of contaminating the insertable part of the catheter prior to insertion. It may also be used to manipulate the catheter during and after withdrawal, thereby avoiding the risk of the user being contaminated by the used catheter. Thus, after withdrawal of the insertion aid from the catheter the insertion aid is usable as an insertion aid for maneuvering the catheter during insertion into the human cavity.

Further, the insertion aid will always be kept securely in place before it is removed and used as an insertion aid. At the same time, if the user does not need an insertion aid for manipulation of the catheter, the insertion aid can be allowed to remain in its non-released state during the entire use.

The insertion aid is further easy to remove, and thereafter to move along the length of the elongate shaft, thereby facilitating contamination free handling of the catheter, which reduces the risk of urinary tract infections and the like.

In addition, the tubular insertion aid is easy and cost-efficient to produce, e.g. by injection molding, and also is easy to mount on the catheter during manufacturing, both manually and automatically.

The insertion aid is preferably sufficiently flexible to be compressed, e.g. by applying a pressure between the thumb and the index finger, over the elongate shaft. Sufficient flexibility of the insertion aid may be accomplished by forming it by a sufficiently flexible material, and/or by having a sufficiently narrow thickness. Further, the thickness may vary over the tubular part, thereby making some parts more flexible than others. Preferably, the tubular part has a Shore A hardness in the range 40-80, and preferably in the range 50-70, and most preferably in the range 55-65.

The tubular insertion aid can e.g. be manufactured by injection molding.

### Brake

The urinary catheter assembly may further comprise a braking element 7 arranged in the vicinity of the openable end, the braking element 7 being arranged inside the tubular casing 2 and allowing temporary fixation of the catheter shaft 11 by manual compression of the braking element 7 through walls of the tubular casing 2. The braking element 7 is of particular advantage in combination with a tubular insertion aid, since it allows a simple and effective way of retaining the catheter during movement and repositioning of the tubular insertion aid. However, the braking element is of great use also without a tubular insertion aid, such as in manipulation of the catheter by directly touching it or through the walls of the package. In addition, the braking element may also be used instead of, or in conjunction with, a one-way locking element, as discussed above, to maintain the proximal part of the catheter and the proximal part of the package together e.g. during extension of an extendible package.

In one embodiment, as shown in Fig. 1 and as illustrated with further detail in Fig. 10, the braking element 7 may comprise at least one part 71 with a solid circumference embracing the catheter shaft 11, forming a cylindrical, tubular part. The braking element 7 may further comprise at least one arm 72 extending out from the solid part 71 in a longitudinal direction of the catheter, and parallel to the catheter shaft. Thus, the arms extend freely, presenting free ends, along the catheter shaft towards one direction, and preferably in the distal direction, directed towards the distal end of the catheter. Preferably, at least two arms are provided, such as two, three, four or five arms. The arms are preferably equidistantly separated around the circumference.

In the illustrative example, two braking arms 72 are provided, arranged on opposite sides of the catheter shaft. However, there may also e.g. be four arms, arranged at 90 degrees angular distance around the circumference, or six arms, arranged at 60 degrees angular distance around the circumference.

The arms are arranged to provide a greatly increased friction to the catheter shaft when squeezed into compression onto the catheter shaft, thereby prohibiting movement of the catheter shaft when braked by the braking elements. To enhance this effect, the braking arms may be provided with e.g. a surface texture providing an increased friction, such as a corrugation, embossment, etc. In a preferred embodiment, the braking arms are provided with sharp protrusions, extending inwardly towards the catheter shaft, and preferably being arranged at or in the vicinity of the free end(s). Hereby, the friction between the arms and the catheter shaft is increased, and the compression force needed to arrest the catheter shaft is reduced. Thus, the arms may be provided with pinch surfaces to provide an increased friction towards the catheter shaft. However, the arms are preferably arranged and structured so as not to damage the catheter, and in particular not to damage a hydrophilic coating arranged on the catheter shaft.

At least the arms, and preferably also the part with the solid circumference, are made of a flexible, relatively soft material, that can easily be squeezed into engagement with the catheter shaft, but gentle enough not to damage the hydrophilic surface coating of the catheter. Thus, at least the braking arms, and preferably the entire braking element, can be brought into engagement with the catheter shaft upon application of a moderate finger pressure to the outer surface of the element, and through the wall of the package.

Thus, each braking arm may act as a lever arm, functioning as a rigid or semi-rigid bar or beam that is free to pivot, bend, flex or the like around a fixed point or moment in order to take advantage of the mechanical force produced by the moment action. Thus, the arms may be, for example, made of a substantially rigid or semi-rigid material, such as a rigid polymer, laminated material, or the like. Some examples of suitable rigid polymers of the invention include polypropylene, polyethylene, polycarbonate, or the like. Further, rigidity or flexibility can be adjusted by the thickness of the material selected, design factors, such as cross beams, as well as other structural support means known in the art.

Thus, the braking arms forms clamping members, connected by a pivot joint to the part with the solid circumference.

The braking arms are preferably biased not to be in contact with the catheter shaft when not compressed, thereby allowing free movement of the catheter when the braking arms are not deliberately brought into a squeezed, arresting state.

The braking element, including the part with the solid circumference and the braking arms, are preferably formed together, e.g. by injection molding.

The length of the part forming a solid circumference may be in the range of 0.5-10 cm, and preferably within the range of 1-5 cm, and most preferably in the range of 1-3 cm. The braking arms preferably have a length within the range of 0.5-10 cm, and preferably within the range of 1-7 cm. and most preferably in the range of 2-5 cm.

The braking element is preferably arranged with an inner diameter providing a clearance toward the catheter shaft, i.e. presenting an inner diameter that is significantly larger than an outer, external diameter of the catheter shaft. Hereby, the braking element does not in any way restrict movement of the catheter shaft when no external pressure is applied towards the braking element.

During use, the braking element is used to brake and arrest the catheter during repositioning of the insertion aid, extension of the package, and the like, and is then released to allow movement of the catheter in any desired direction.

### Annular package

In an embodiment of the urinary catheter assembly, the tubular casing 2 forms an annular package, and defining a cavity therein for accommodating at least the insertable part of the catheter, and preferably the entire catheter. The cavity is preferably sterile and moisture proof. To this end, the package may e.g. be made of a fluid impermeable material. The package is preferably, at least in most parts, made of a relatively flexible material. The package may be transparent, but may alternatively be opaque or semi-opaque.

The package is preferably in the form of a tube or hose, and in a preferred embodiment, the package is arranged to form an annular shape, with the cavity extending as an annular cavity inside the package. The annular package may be provided with a breakable or openable member, allowing two ends of the annular package to be separated from each other, for opening of the package upon use. The annular cavity may be closed in the opening area, forming a generally C-shaped annular cavity. However, the annular cavity may also be open in the opening area, forming a generally O-shaped annular cavity. The annular package embodiment will be discussed in further detail below.

The package may be openable by having two separable ends attached together. In the illustrative example of Fig. 1, the distal end of the package is inseparably connected to the cap 5, whereas the proximal end of the package is separably connected to the cap. Thus, by loosening the cap from the proximal end of the package, the tubular casing is opened.

At the proximal end, the proximal part of the catheter is arranged so that it extend past the brake7, abutment 21 and the one-way locking arrangement 4. A part of the proximal part of the catheter preferably extends past the interface between the cap 5 and the proximal part of the package. Hereby, when the cap 5 is released from the proximal end of the package, a part of the proximal part of the catheter will protrude from the proximal part, and from the one-way locking element. This protruding part may, in the closed disposition, be stabilized by the distal end of the package, and e.g. by being inserted into a sleeve 8 having a relatively narrow opening for accommodation of the catheter tip/catheter shaft. Hereby, the catheter shaft, an in particular the insertion tip, becomes better protected.

Even though the package is here formed in an annular shape, the internal cavity inside the package, for housing the catheter, need not necessarily be annular. Instead, the cavity may e.g. be closed at one or more positions, thereby defining e.g. one or several separations along the extension of the annular package. For example, the cavity may be generally C-shaped, with a closing at or in the vicinity of the cap 5, so that there is no fluid communication allowed through the opening area.

However, the cavity within the annular, tubular package may also be annular, i.e. with flow communication possible around the entire, generally O-shaped cavity. This alternative is generally preferred, in particular if the catheter is a hydrophilic catheter, and where the tubular casing also encloses a wetting fluid for activation of the hydrophilic surface/coating, since it allows the wetting fluid to distribute very efficiently within the entire cavity, there ensuring an adequate wetting of the catheter.

Another advantage with the cavity encircling the whole package is that the opening of the tubular casing is facilitated, since it only need to be opened once, at one position, to provide two opened ends of the tubular casing, for use as an catheter extension during urine drainage, as discussed in the foregoing.

As shown in the illustrative embodiment, the package generally presents two relatively straight parts 22c, 22d, connected together by two curved parts 22a, 22b. The straight parts may be arranged to enclose and accommodate the rearward part and the forward part of the catheter, whereas one of the curved parts 22b may be arranged to accommodate an intermediate part of the catheter interconnecting the forward and rearward parts. The second curved part, 22a, may be essentially empty.

The curved part 22b arranged to accommodate the intermediate part of the catheter is preferably provided with a significantly higher radius of curvature than the other curved part 22a. Hereby, the curved part in which the catheter is arranged provides a gentle, smooth transition for the catheter, avoiding risk of damaging the catheter during storage, such as due to kinking, permanent deformations etc. The other, empty curved part may on the other hand present a much sharper curvature. Hereby, the length of the non-filled curved part 22a is also preferably much shorter than the length of the filled curved part 22b. Hereby, the straight parts 22c and 22d are preferably non-parallel to each other, but arranged at an angle towards each other, pointing the parts towards each other and to a position close to the non-filled curved part 22a.

As discussed in the foregoing, the curved parts 22a, b, may be use as extendible sections, and may e.g. be formed by corrugated, bellow-like folded walls.

The cap may be interconnected with the proximal part of the tubular casing in various ways, to provide an easy-to-open mechanism, also for users having poor dexterity, etc. For example, the cap and the proximal part of the tubular casing may be connected together by interlocking elements, such as snap-fit elements, a threaded coupling, a friction fit, etc.

In an embodiment, the interlocking elements are in form of a threaded coupling. The thread may be short so that the user only has to turn the handle and connector about 90 degrees or even less, such as 60 degrees, to disconnect the cap from the proximal part of the package. The threaded coupling has the advantage that it is easy to use even for users having poor hand dexterity.

In another embodiment, the interlocking elements are provided as first snap-fit element on the cap and second snap-fit elements on the proximal part of the package, the first and second snap-fit elements being complementary snap-fit elements. This is an easy and well-functioning way of making the cap and the proximal package part detachably attached to each other. The snap-fitting elements may be provided as an annular and radially inwardly extending ridge inside the cap adapted for cooperation with an annular groove on the outer surface of the proximal package part.

The interlocking elements are preferably arranged to maintain a closed and sterile closure for the catheter during storage, and to enable simple opening of the package when it is to be used. In a preferred embodiment, the interlocking elements also enables re-closing of the tubular package after use, so that the used catheter can be re-inserted into the package after use, and the tubular casing then be closed to a closed state again, preventing remaining fluid etc to escape the package.

### Concluding remarks

Specific embodiments of the invention have now been described. However, several alternatives are possible, as would be apparent for someone skilled in the art. For example, although the wetting fluid in the described embodiments is arranged in direct contact with the catheter, it may alternatively be arranged separated from the catheter, in a wetting fluid container.

In particular, the features disclosed above, such as the annular package, the one-way locking, the drip stop valve, the braking element, the extendible package, etc, may all be used in combination, but may alternatively be used in combination with other types of embodiments, individually, in any pairwise combination, or in combinations of three or more of these features.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A urinary catheter assembly comprising:
a urinary catheter having a proximal part with an insertion tip, a distal part and a tubular shaft extending there between, wherein the distal part, on a least a part thereof, has a greater cross-sectional dimension than the tubular shaft and the proximal part;
a tubular casing being adapted to be arranged in a first storage position and a second use position and with an openable distal end, wherein in the first storage position, the tubular casing is arranged to accommodate at least the insertable part of the urinary catheter, in which the tubular casing forms a sealed and closed compartment around the insertable part of the urinary catheter, and wherein in the second use position, the openable end of the tubular casing is opened, allowing the insertable part of the urinary catheter to be expelled out from the tubular casing, wherein the tubular casing, in a proximal part thereof, is provided with a narrowing section, providing a decreased internal diameter compared to at least an adjacent, more distal part of the tubular casing, the narrowing section forming a stop abutment for the distal part of the urinary catheter; and
a tubular insertion aid being detachable connected to the tubular casing, and arranged in the vicinity of the openable end of the tubular casing;
wherein the tubular casing is further provided with a braking element arranged in the vicinity of the openable end, the braking element being arranged inside the tubular casing and allowing temporary fixation of the catheter shaft by compression of the braking element through walls of the tubular casing.

2. The urinary catheter assembly of claim 1, wherein the braking element comprises at least one braking arm extending in parallel with the catheter shaft, and with a free end.

3. The urinary catheter assembly of claim 2, wherein the at least one braking arm comprises a plurality of braking arms being separately arranged around the circumference of the catheter shaft.

4. The urinary catheter assembly of claim 2 or 3, wherein the braking arms are provided with an inward sharp protrusion facing the catheter shaft, and being arranged at or in the vicinity of the free end.

5. The urinary catheter assembly of any one of the preceding claims, wherein the tubular insertion aid is arranged exteriorly on the tubular casing.

6. The urinary catheter assembly of any one of the preceding claims, wherein, in the first storage position, the tubular insertion aid is arranged overlying the braking element.

7. The urinary catheter assembly of any one of the preceding claims, wherein the tubular insertion aid has funnel shape, whereby the proximal end has a smaller cross-sectional area than the distal end.

8. The urinary catheter assembly of any one of the preceding claims, wherein the tubular casing comprises two open ends being connected together for closure, thereby forming an annular, closed cavity within the tubular casing in the storage position.

9. The urinary catheter assembly of any one of the preceding claims, wherein a drip stop valve is arranged at or in the vicinity of the distal part of the catheter, the drip stop valve being arranged to allow liquid to flow through the drip stop valve in a direction from the proximal part to the distal part when the fluid pressure exceeds a threshold value, and to stop flow in said direction when the fluid pressure does not exceed said threshold value.

10. The urinary catheter assembly of any one of the preceding claims, wherein the tubular casing is extendible between a relatively shorter length in the first storage position, and a relatively longer length in the second use position.

11. The urinary catheter assembly of any one of the preceding claims, at least one cap arranged to close at least one openable end of the tubular casing in the first storage position and to be openable by an opening action.

12. The urinary catheter assembly of claim 11, further comprising a one-way locking element arranged in the vicinity of the openable end of the tubular casing, and arranged to brought into a one-way locked disposition to lock the catheter shaft for movement in one direction in relation to a proximal part of the tubular casing when said cap is opened by said opening action, said one-way locking disposition preventing displacement of the urinary catheter away from the proximal end of the tubular casing in a first direction towards the distal end but allowing displacement of the urinary catheter in an opposite second direction, and further arranged automatically to release the one-way locking disposition upon movement of the catheter in the second direction.

13. The urinary catheter assembly of any one of the preceding claims, wherein the catheter is a hydrophilic catheter, and wherein the assembly further comprises a wetting fluid for activation of the hydrophilic catheter.

14. The urinary catheter assembly of any one of the preceding claims, wherein the distal part of the catheter comprises a flared connector.

15. The urinary catheter assembly of any one of the preceding claims, wherein the casing in the first closed position provides a sterile and moisture proof compartment for the insertable section of the catheter in the closed storage position.
